(19) Europäisches Patentamt
European Patent Office
Office européen des brevets

(11) **EP 4 027 872 B1**

(12) **EUROPEAN PATENT SPECIFICATION**

(45) Date of publication and mention of the grant of the patent:
**18.06.2025 Bulletin 2025/25**

(21) Application number: **20862130.0**

(22) Date of filing: **10.09.2020**

(51) International Patent Classification (IPC):
*A61B 5/02* (2006.01)          *A61B 5/1455* (2006.01)
*A61B 5/026* (2006.01)          *A61B 5/024* (2006.01)
*A61B 5/03* (2006.01)

(52) Cooperative Patent Classification (CPC):
**A61B 5/031; A61B 5/0082;** A61B 5/02125;
A61B 5/6815; A61B 5/6823; A61B 5/6824;
A61B 5/6826; A61B 5/6828; A61B 5/6829

(86) International application number:
**PCT/US2020/050166**

(87) International publication number:
**WO 2021/050703 (18.03.2021 Gazette 2021/11)**

(54) **A SYSTEM FOR NON-INVASIVELY MEASURING BLOOD VOLUME OSCILLATIONS INSIDE CRANIUM OF A HUMAN SUBJECT**

SYSTEM ZUM NICHTINVASIVEN MESSEN VON BLUTVOLUMENSCHWINGUNGEN IM SCHÄDEL EINES MENSCHEN

SYSTÈME DE MESURE NON INVASIVE DES OSCILLATIONS DU VOLUME SANGUIN À L'INTÉRIEUR DU CRÂNE D'UN SUJET HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2019 US 201962973021 P**

(43) Date of publication of application:
**20.07.2022 Bulletin 2022/29**

(73) Proprietor: **Vivonics, Inc.**
**Bedford MA 01730 (US)**

(72) Inventors:
• **PIERRO, Michele**
**Wesford, MA 01886 (US)**
• **HIRSCHMAN, Gordon, B.**
**Cohoes, NY 12047-1440 (US)**

(74) Representative: **Dolleymores**
**9 Rickmansworth Road**
**Watford, Hertfordshire WD18 0JU (GB)**

(56) References cited:
WO-A1-2019/040849          US-A1- 2006 074 283
US-A1- 2009 234 245          US-A1- 2012 108 927
US-A1- 2015 018 697          US-A1- 2016 278 643
US-A1- 2018 110 449          US-A1- 2019 021 646
US-A1- 2019 269 853

• **RUWAN A WEERAKKODY ET AL: "Slow vasogenic fluctuations of intracranial pressure and cerebral near infrared spectroscopy-an observational study", ACTA NEUROCHIRURGICA ; THE EUROPEAN JOURNAL OF NEUROSURGERY, SPRINGER-VERLAG, VI, vol. 152, no. 10, 11 August 2010 (2010-08-11), pages 1763 - 1769, XP019853158, ISSN: 0942-0940**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

RELATED APPLICATIONS

This application claims benefit of and priority to U.S. Provisional Application Serial No. 62/973,021 filed September 12, 2019, under 35 U.S.C. §§119, 120, 363, 365, and 37 C.F.R. §1.55 and §1.78.

GOVERNMENT RIGHTS

This invention was made with U.S. Government support under Contract No. W81XWH-17-C-0006, awarded by the US Army. The Government has certain rights in the invention.

FIELD OF THE INVENTION

This invention relates to a system and method for non-invasively measuring blood volume oscillations inside the cranium of a human subject and using the measured oscillations to non-invasively determine intracranial pressure (IPC).

BACKGROUND OF THE INVENTION

Conventional optical sensing systems, particularly those using near-infrared (NIR) light, have been used to monitor both blood volume oscillations and blood oxygen content by using one or more light sources and detector placed on the skin. All such measurements that seek to obtain information from within the cranium are subject to confounding of the reflected (or scattered) return light signal to the detector by the necessity of the light to transit the superficial region or superficial space above the cranium,. In conventional functional near infrared spectroscopy (fNIRS) applications, the primary objective is to detect changes in oxygenation attributed to regional brain activity. Conventional systems and methods have been developed and proposed for removal of the superficial contribution to the cranial signal in such applications, However, such conventional systems and methods typically apply filtering techniques that are designed to remove cardiac-driven oscillations from the data in order to retrieve the slower activation-driven oxygen changes, For determination of blood volume oscillations within the cranium, such filtering may not be applied because the filtering would distort the shape and timing of the cranial blood volume waveforms, which may be required for other processing, such as non-invasively deterraining intra-cranial pressure (ICP) based on the impact of ICP on the pulse wave velocity inside the cranium. Therefore, such conventional systems and methods may be unable to accurately measure blood volume oscillations inside the cranium or non-invasively determine ICP.

Thus, there is a need for a non-invasive system and method to measure blood volume oscillations inside the cranium of a human subject that removes contributions of photon absorption and scattering in the superficial layer or superficial space from the detected light from inside the cranium which has passed through the superficial space, in order to accurately measure blood volume oscillations inside the cranium without significantly distorting wave shape or timing. There is also a need for such a system and method to measure blood volume oscillations inside the cranium of a human subject and use the measured blood volume oscillation to non-invasively measure ICP.

U.S. Pub. No. 2019/0269853 discloses a system that determines fluid administration based on fluid responsiveness to regional oxygen saturation of a subject. The subject may receive the fluid responsiveness and regional oxygen saturation from an external source and may be determined one or both based on the received physiological signals U.S. Pub. No. 2019/0021646 discloses a system for non-invasively measuring oxygen wavelength dependent changes in optical absorptions of brain tissue. The brain tissue is trans-cranially illuminated by light sources in low-absorption spectrum windows for the tissue in the visible or near infrared parts of the spectrum. U.S. Pub. No. 2012/0108927 discloses a method and apparatus for non-invasively determining blood oxygenation within a subject tissue that utilizes near infrared spectrophotometric (NIRs) sensor capable of transmitting a light signal into the tissue of a subject and sensing the light signal once it has passed through the tissue. WO 2019/040849 discloses an optical probe including three optical elements including at least one light source and at least one light detector. The three optical elements are positioned in a triangular configuration. A light shroud is disposed radially around the exposed distal and portions of the optical fibers coupled to the light source. Weerakkody et. al. discloses slow vasogenic fluctuation of intercranial pressure and cerebral using near infrared spectroscopy. U.S. PbB. No. 2018/0110449 teaches an oxygen saturation measuring sensor which includes a pad attachable to the human body, a plurality of light sources arranged on the pad and irradiate near-infrared light. A plurality of light receiving elements arrange on the pad to be adjacent in a separated stage and correspond to one of the pluralities of light sources with reference to a common center. U.S. Pub No. 2015/0018697 disclosed a system with a first sensor placed proximate to a perfusion field of an artery receiving blood emanating from the cranial cavity and monitors pulsation of the artery receiving blood emanating from the cranial cavity. A second sensor is placed proximate to a perfusion field of an artery which does not receive blood emanating from the cranial cavity. The second sensor monitors pulsation of the artery which does not receive blood and emanating from the cranial cavity. A third sensor is placed distally from a heart and monitors pulsation of a distal artery. A processing subsystem responsive to signal from the first, second and third sensor determines intracranial pressure. U.S. Pub No. 2006/0074283 discloses an apparatus from monitoring vital sign parameters in a biological entity. The apparatus includes at least one light source which transmits light through the biological entity and at least one photodetector for receiving light transmitted through the biological entity A signal is generated and response to transmitted or reflected light through the biological entity signal which corresponds to at least one characteristic of the generally unimpeded blood flow through the biological entity. U.S. PUB No. 2016/0278643 teaches a signal derived from the brain attribute cortex maybe may be extracted by separating and removing influence of the skin blood flood included in NIRs signals. A biphotonic measurement apparatus is used to separate signals simultaneously measured with a plurality of irradiator-detector distances into the brain blood flood-derived signals and skin blood flow-derived signals using SD distance dependency of signal amplitude. U.S. Pub No. 2009/0234245 discloses a system for a noninvasive detection of intercranial pressure (ICP) variation in an intracranial compartment of a patient. Optical radiation is propagated trans-cranially into the cranial compartment and optical radiation that has migrated through at least a portion of the cranial compartment and back out of the cranium is detected.

## SUMMARY OF THE INVENTION

According to the present inventions there is provided a system for non-invasively measuring blood volume oscillations inside a cranium of a human subject, the system comprising:

a first light source adapted to be placed on the skin above the cranium of the human subject configured to emit light which penetrates a superficial space outside the cranium;

a detector adapted to be placed on the skin above the cranium of the human subject spaced from the first light source by a first predetermined separation distance that causes the detector to detect light which reflects from the superficial space outside the cranium and output superficial output signals; wherein

a second light source adapted be placed on the skin above the cranium configured to emit light which penetrates through the superficial space outside the cranium to inside the cranium and the detector is spaced from the second light source by a second predetermined separation distance that causes the detector to detect light which reflects from inside the cranium and output cranial output signals;

a processing subsystem coupled to the first light source, the second light source, and the detector, the processing subsystem configured to alternately enable the first light source and the second light source and alternately enable the detector to detect the light which reflects from the superficial space outside the cranium and the light which reflects from inside the cranium to generate the superficial output signals and cranial output signals, the processing subsystem responsive to the superficial output signals and the cranial output signals and further configured to reduce contributions from the superficial space existing in the cranial output signals and generate corrected cranial output signals indicative of blood volume oscillations inside the cranium; and

a reference sensor adapted to be placed on the human subject and coupled to the processing subsystem, the reference sensor configured to provide reference signals for calculating a differential pulse transit time, a phase shift, or transfer function from one location on the human subject to the corrected cranial output signals.

Further aspects are defined in the dependent claims.

## BRIEF DESCRIPTION OF THE SEVERAL VIEWS OF THE DRAWINGS

Other objects, features and advantages will occur to those skilled in the art from the following description of a preferred embodiment and the accompanying drawings, in which:

Fig. 1 is a schematic block diagram showing primary components of one example of the system and method thereof for non-invasively measuring blood volume oscillations inside the cranium of a human subject and determining intracranial pressure;

Fig. 2 is a schematic block diagram showing in further detail one example of the placement of the sensors and detector on the cranium shown in Fig. 1;

Fig. 3 is a schematic block diagram showing example locations for the placement of the detector shown in Figs. 1 and 2;

Fig. 4 is a schematic block diagram showing the primary components of another embodiment of the system and method thereof for non-invasively measuring blood volume oscillations inside the cranium of a human subject and determining intracranial pressure;

Fig. 5 is a schematic block diagram showing in further detail one example of the placement of the sensors and detectors shown in Fig. 4;

Figs. 6A-6D show additional examples of the placement of the sensors and detectors shown in one or more of Pigs, 1-5;

Fig. 7 is a flowchart showing one example of the method for non-invasively measuring blood volume oscillations inside the cranium of a human subject and determining intracranial pressure; and,

Fig. 8 is a flowchart showing one example of the primary steps for the system and method shown in one or more of Figs. 1-7 used to determine ICP,

DETAILED DESCRIPTION OF THE INVENTION

Aside from the preferred embodiment or embodiments disclosed below, this invention is capable of other embodiments and of being practiced or being carried out in various ways. Thus, it is to be understood that the invention is not limited in its application to the details of construction and the arrangements of components set forth in the following description or illustrated in the drawings. If only one embodiment is described herein, the claims hereof are not to be limited to that embodiment, Moreover, the claims hereof are not to be read restrictively unless there is clear and convincing evidence manifesting a certain exclusion, restriction, or disclaimer.

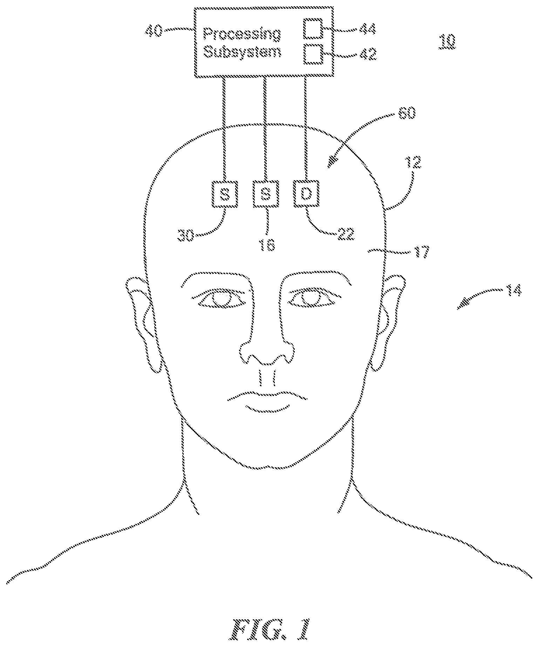

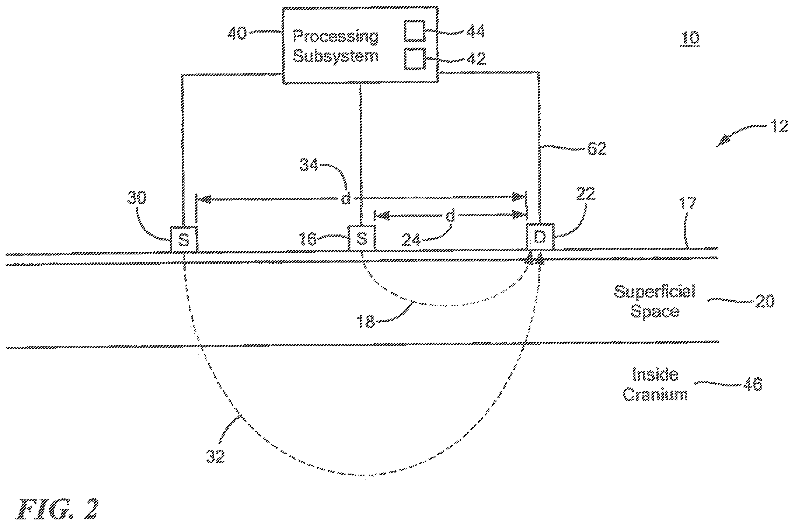

There is shown in Fig. 1 one embodiment of system 10 for non-invasively measuring blood volume oscillations inside cranium 12 of human subject 14, System 10 includes first light source 16 adapted to be placed on skin 17 above cranium 12 of human subject 14. First light source 16 is configured to emit light 18, Fig. 2, which penetrates superficial space 20 outside cranium 12.

System 10, Figs, 1 and 2, also includes detector 22 adapted to be placed on skin 17 above cranium 12 of human subject 14. Detector 22 is spaced from first light source 16 by first predetermined separation, distance d-24, Fig. 2, that causes detector 22 to detect light 18 which reflects from superficial space 20 outside cranium 12 and output superficial output signals.

System 10, Figs. 1 and 2, also includes second light source 30 adapted to be placed on skin 17 above cranium 12. Second light source 30 is configured to emit light 32, Fig. 2, which penetrates through superficial space 20 outside cranium 12 to inside cranium 46. Detector 22 is spaced from second light source 30 by second predetermined separation distance, d-34, that causes detector 22 to detect light 32 which reflects from inside cranium 46 and output cranial output signals. In one example, first light source 16, second light source 20 and detector 22 may be adapted to be placed on forehead 60, Fig. 1, of cranium 12 as shown.

System 10, Figs. 1 and 2, also includes processing subsystem 40 coupled to first light source 16, second light source 30, and detector 22 as shown. Processing subsystem 40 may be a processor, e.g., processor 42, one or more processors, an application-specific integrated circuit (ASIC), firmware, hardware, and/or software (including firmware, resident software, micro-code, and the like) or a combination of both hardware and software. Processing subsystem 40 preferably includes one or more programs stored in a memory, e.g., memory 44, which are configured to be executed by the one or more processors. Computer program code for the programs for carrying out the instructions or operation of processing subsystem 40 may be written in any combination of one or more programming languages, including an object-oriented programming language, e.g., C++, Smalltalk, Java, and the like, or conventional procedural programming languages, such as the "C' programming language, Assembly language or similar programming languages.

Processing subsystem 40 is configured to alternately enable first light source 16 to emit light 18 which penetrates superficial space 20 outside cranium 12 and second light source 30 to emit light 32 which penetrates through the superficial space outside the cranium to inside cranium 46 and alternately enable detector 22 to detect light 18 which reflects from superficial space 20 outside the cranium emitted by first light source 16 to generate the superficial output signals and detect light 32 emitted by second light source 30 which reflects from inside cranium 46 to generate the cranial output signals.

Processing subsystem 40 is responsive to the superficial output signals and the cranial output signals and is further configured to reduce contributions from the superficial space existing in the cranial output signals and generate corrected cranial output signals indicative of blood volume oscillations inside the cranium, Processing subsystem 40 may reduce the superficial contribution in the cranial signal by subtraction of normalized signals, linear regression where the superficial signal is used as a regressor and the unprocessed cranial signal as the regressed, Kalman filtering, other mathematical and statistical techniques known in the art, and physics-based photon propagation models. Preferably, processing by processing subsystem 40 may avoid filtering of frequency components of the signals in the region of about 0.5 Hz to about 2.0 Hz which contains the cardiac signal in order to preserve important wave shape and timing characteristics of the blood volume oscillation in the corrected cranial output signal. In some examples, the correction in the corrected cranial output signals by processing subsystem 40 may be null. As disclosed herein, such uncorrected cranial output signals are still referred to as corrected cranial output signals and the systems and methods discussed below may be utilized but may be less accurate.

The result is system 10 for non-invasively measured blood volume oscillations inside the cranium and has effectively reduced the contributions in the light detected by detector 22 from inside the cranium which has passed through superficial space 20 due to the blood volume oscillations in the superficial space. Thus, system 10 non-invasively, accurately, and effectively measures blood volume oscillations inside the cranium of a human subject. The measured blood volume oscillations may be used to non-invasively, accurately, and effectively measure ICP, as discussed below.

In one design, first light source 16, Figs. 1 and 2, second light source 30, and detector 22 may be adapted to be placed above skin 17 above cranium 12 of human subject 14 with first light source 16 located between second light source 30 and detector 22 as shown.

In one design, first light source 16 and/or second light source 30 may include at least one near-infrared (NIR) light source. In other examples, first light source 16 and/or second light source 30 may include at least one light emitting diode (LED).

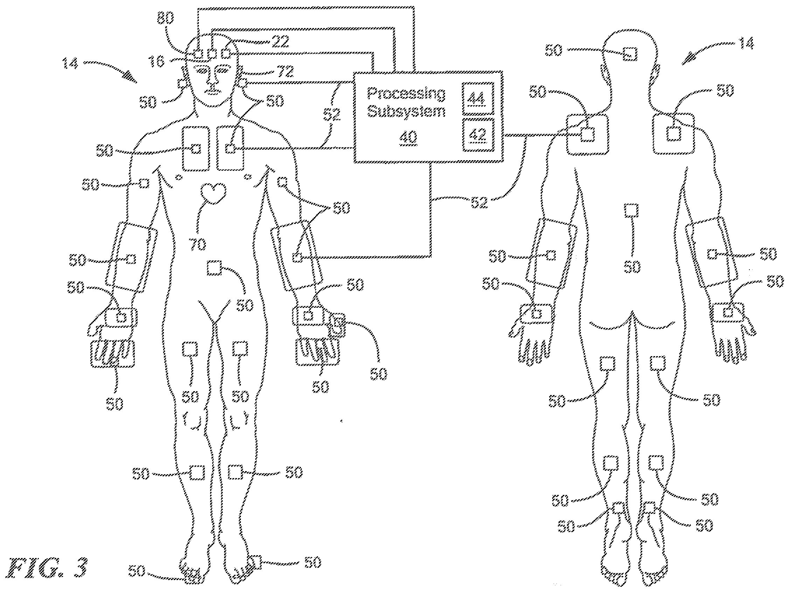

As known by those skilled in the art, pulse transit time is the time it takes for a blood pressure pulse to travel from one location of human subject 14 to another location of human subject 14 in the vasculature of a human subject. If the length along the vascular tree between the two locations on human subject 14 is known, then the pulse wave velocity (PWV) can be calculated from one location on human subject 14 to another location of human subject 14 and used to determine ICP inside the cranium of human subject 14, as discussed below, A differential pulse transit time may also be defined as the time difference between arrival of the cardiac pulse at one location on human subject 14, e.g., any of the locations shown in Fig. 3 (discussed below), to another location on human subject 14, e.g., any of the locations shown in Fig. 3, even though the two locations do not lie on same vascular pathway from the heart, 70, shown in Figure 3.

In one design, system 10 may also include reference sensor 50, Fig. 3, adapted to be placed on human subject 14, e.g., on any of the locations shown in Fig. 3, such as an car, a temple, a finger, a hand, a forearm, a wrist, a chest, a back, a leg, a toe, a foot, and the like, of the human subject. Reference sensor 30 is coupled to processing subsystem 40, exemplarily indicated at 52. Reference sensor 50 is configured to provide reference signals to be used as a time reference for processing subsystem 40 to calculate a differential pulse transit time from one location on human subject 14, e.g., any of the locations shown in Fig. 3, to the corrected cranial output signals.

In one example, reference sensor 30 may include at least one NIR sensor. In another examples, reference sensor 30 may include at least one electrocardiogram (ECG) sensor, at least one plethysmograph sensor, at least one pressure sensor. or similar type sensors.

For example, to non-invasively determine ICP, processing subsystem 40, Figs. 1-3, may be configured to calculate a phase shift of the corrected cranial output signals compared to the reference signals and determine ICP of human subject 14 from the phase difference of the corrected cranial output signals compared to the reference signals.

In another example, processing subsystem 40 may calculate a difference or transfer function between the superficial output signals and the corrected cranial output signals and determine ICP from the difference. In this example, the superficial output signal indicates pulse transit time from the heart along a vascular pathway including the exterior carotid artery and the corrected cranial output signals indicate pulse transit time along a vascular pathway including the interior carotid artery and through the cranium where it is influenced by changes in ICP.

In another example, processing subsystem 40 may calculate a phase shift of the corrected cranial output signals compared to the superficial output signals and determine ICP of human subject 14 from a phase shift of the corrected cranial output signals compared to the superficial output signals.

In one design, processing system 40 may calculate a time delay between the corrected cranial output signals and the superficial output signals and determine intracranial pressure of the human subject from the time delay. There are multiple ways to calculate the time delay or time shift between the corrected cranial output signals and the superficial output signals, e.g., locating a major feature of the superficial output signals and the corrected cranial output signals and calculating a time delay or time shift between the major features. The major features of the superficial output signals and the corrected cranial output signals may include a peak of a waveform of the superficial output signals and a peak of a waveform of the corrected cranial output signals, a trough of a waveform of the superficial output signals and a trough of a waveform corrected cranial output signals, or similar major features of the superficial output signals and the corrected cranial output signals, as known by those skilled in the art.

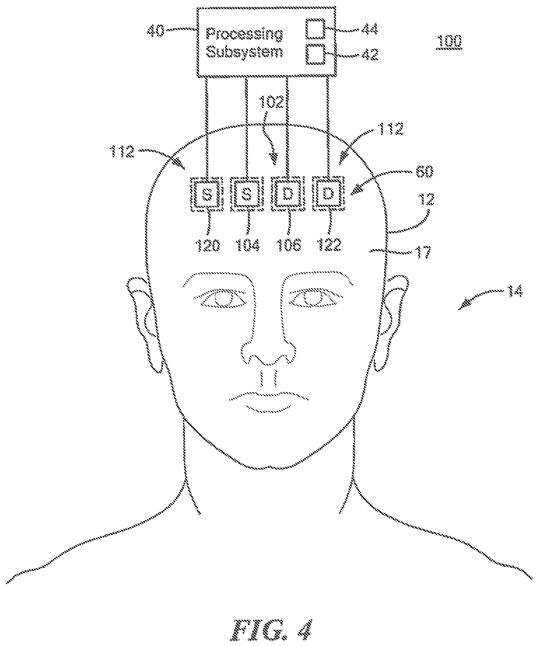

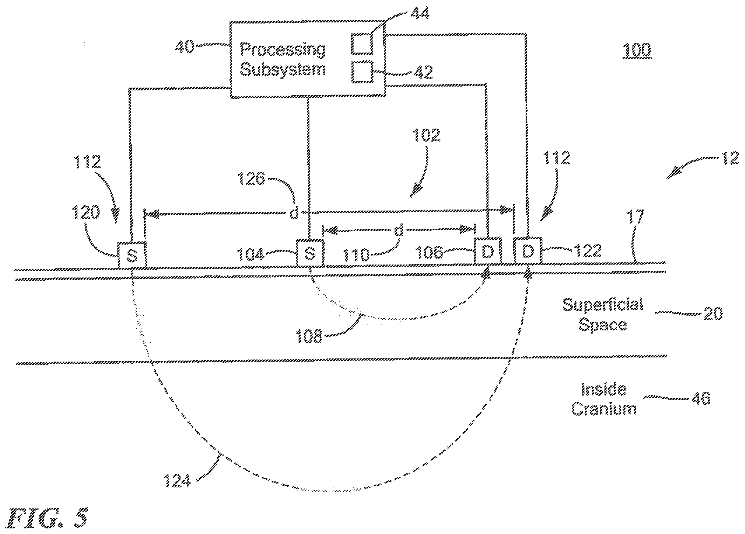

In another embodiment, system 100, Fig. 4, where like parts have been given like numbers, for non-invasively measuring blood volume oscillations inside cranium 12 of human subject 14 includes first sensor 102 adapted to be placed on skin 17 above cranium 12 of human subject 14. First sensor 102 includes first light source 104 and first light detector 106. First light source 104 is configured to emit light 108, Fig. 5, which penetrates superficial space 20 outside cranium 12. First detector 106 is spaced from first light source 104 by first predetermined separation distance, d-1 10, that causes first detector 106 to detect light 108 which reflects from superficial space 20 outside cranium 12 and output superficial output signals.

System 100 also includes second sensor 112 adapted to be placed on skin 17 above cranium 12 of human subject 14. Preferably second sensor 112 is adapted to be placed proximate to first sensor 102, Second sensor 112 includes second light source 120 and second detector 122. Second light source 120 is configured to emit light 124 which penetrates through superficial space 20 outside cranium 12 to inside cranium 46, Second detector 122 is preferably placed proximate first detector 106 as shown in Figs. 4 and 5 and is spaced from second light source 120 by second predetermined separation distance, d-126, Fig. 5, that causes detector 122 to detect light 124 which reflects from inside cranium 146 and output cranial output signals.

Processing subsystem 40, Figs. 4 and 5, is coupled to first sensor 102 including first light source 104 and first detector 106 and second sensor 112 including second light source 120 and second detector 122. Processing subsystem 40 is configured to alternately enable first light source 104 to emit light 108 which penetrates superficial space 20 outside cranium 12 and second light source 120 to emit light 124 which penetrates through the superficial space outside the cranium to inside cranium 46 and alternately enable first detector 106 to detect light 108 which reflects from superficial space 20 outside cranium 12 and alternately enable second detector 122 to detect light 124 which reflects from inside cranium 46 to generate the superficial output signals and the cranial output signals. Processing subsystem 40 is responsive to the superficial output signals and the cranial output signals and is configured to reduce contributions from the superficial space existing in the cranial output signals and generate corrected cranial output signals indicative of blood volume oscillations inside the cranium, similar as discussed above with reference to one or more of Figs. 1-3.

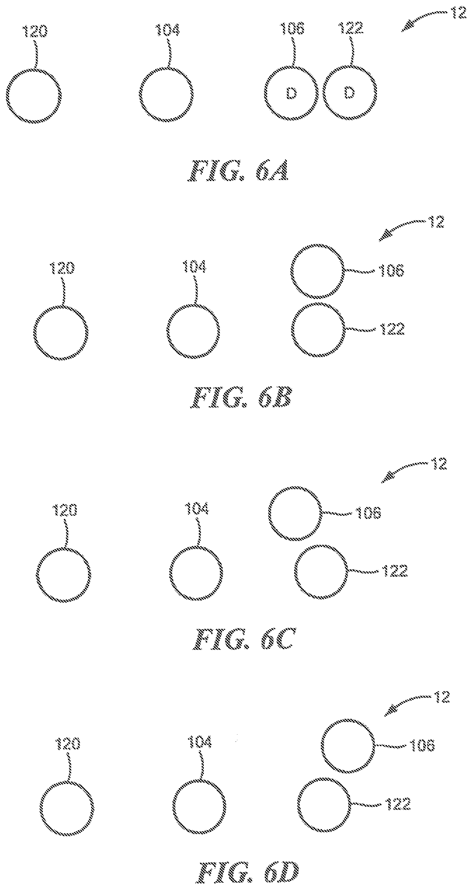

In one design, first sensor 102, Figs. 4 and 5, and second sensor 112 are adapted to be placed above skin 17 of cranium 12 with first light source 104 preferably located between second light source 120 and first detector 106 and second detector 122, e.g. in a line as shown in a top view in Fig 6A. In other examples, first detector 106 and second detector 122 may be adapted to be placed in a side-by-side configuration from first light source 104 and second light source 120, e.g., as shown in top view Fig 6B, or at an angle to each other, e.g. as shown in Figs. 6C and 6D.

in one example, first sensor 102 and/or second sensor 112 may include at least one NIR sensor. In one example, first light source 104 and/or second light source 122 may include at least one light emitting diode (LED).

In one embodiment, first sensor 102 and second sensor 112 may be configured to share first detector 106 or second detector 122, e.g. similar as discussed above with reference to Figs. 1-3.

System 100, Figs. 4 and 5, may also include third sensor 50, similar as discussed above with reference to Fig. 3 adapted to be placed on human subject 14 configured to provide reference signals to be used as a time reference for calculating a pulse transit time from one location on the human subject to the cranial output signals, similar as discussed above with reference to one or more of Figs, 1-3.

In one example, first sensor 102, Figs. 4 and 5, and second sensor 112 may be adapted to be placed on forehead 60, Fig. 4, of cranium 12. Similarly, as discussed above, third sensor 30 may be adapted to be placed on any location of human subject 14 as discussed above with reference to Fig. 3, In one example, third sensor may be adapted to be placed approximately the same distance from heart 70, Fig. 3, as first sensor 102 and/or second sensor 112.

Processing subsystem 40, Figs, 4 and 5, may also be configured to calculate a phase shift of the corrected cranial output signals compared to the reference signals and determine ICP of human subject 14 from the phase shift of the corrected cranial output signals compared to the reference signals.

Similar as discussed above with reference to Figs 1-3, processing subsystem 40, Figs. 4 and 5, may also be configured to calculate a difference or transfer function between the superficial output signals and the corrected cranial output signals and determine ICP from the difference or transfer function.

In another embodiment, processing subsystem 40, Figs. 4 and 5, may be configured to calculate a phase shift of the corrected cranial output signals compared to the superficial signals and determine ICP of human subject 14 from the phase shift of the corrected cranial output signals compared to the superficial signals.

In one design, processing system 40 may calculate a time delay between the corrected cranial output signals and the superficial output signals and determine intracranial pressure of the human subject from the time delay, similar as discussed above.

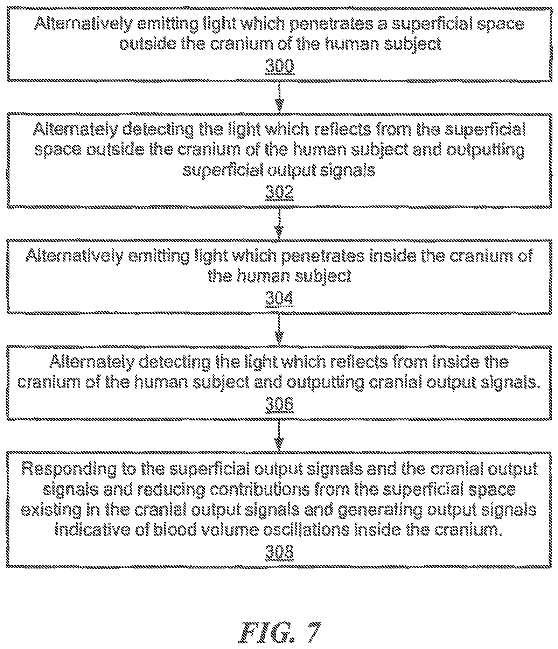

In one embodiment, the method for non-invasively measuring blood volume oscillations inside the cranium of a human subject may include alternately emitting light which penetrates a superficial space outside the cranium, step 300, Fig. 7. The method also includes alternately detecting light which reflects from the superficial space outside the cranium of the human subject and outputting superficial output signals, step 302. The method also includes alternately emitting light which penetrates inside the cranium of the human subject, step 304, The method also includes alternately detecting the light which reflects from inside the cranium of the human subject and outputting cranial output signals, step 306, The method also includes responding to the superficial output signals and the cranial output signals and reducing contributions from the superficial space existing in the cranial output signals and generating corrected cranial output signals indicative of blood volume oscillations inside the cranium, step 308, similar as discussed above with reference to one or more of Figs. 1-6.

In one example, the method may also include providing reference signals to be used as a time reference for calculating a pulse transmit time from one location of a human subject to the corrected cranial output signals.

The method may include calculating a phase shift in the corrected cranial output signals compared to the reference signals and determining intracranial pressure of the human subject from the phase shift of the corrected cranial output signals compared to the reference signals.

The method may include calculating a difference or transfer function between the superficial output signals and corrected cranial output signals and determining intracranial pressure from the difference or transfer function.

The method may include calculating a phase shift of the corrected cranial output signals compared to the superficial output signals and determining intracranial pressure of the human subject from the phase shift of the corrected cranial output signals compared to the superficial output signals.

The method may include calculating a magnitude of the superficial output signals and the corrected cranial output signals and determining intracranial pressure of the human subject from a difference in magnitude of the superficial output signals and the corrected cranial output signals.

In one design, the method may include calculating a time delay between the corrected cranial output signals and the superficial output signals and determining intracranial pressure of the human subject from the time delay.

In an alternate embodiment, system 10, 100, and the method thereof discussed above with reference to one or more of Figs. 1-8 may utilize one or more or multiple light sources and one or more or multiple detectors adapted to be placed on the forehead of the human subject provided the combination of at least one light source and one detector are separated by a separation distance that causes the detector to receive light that reflects from the superficial space above the cranium of the human subject to provide the superficial signals and at least one light source and one detector are separated by a separation distance that causes the detector to receive light that reflects from the cranium of the human subject to provide cranial output signals. When such one or more light sources and one or more detectors are employed there may be multiple light source/detector pairings that provide the desired signals but from different positions over the cranium, which may be desirable to provide a diversity of locations where the measurement is made in order to further confirm the measurement or improve its accuracy or reliability by combining measurements from multiple locations, for example by averaging the measurements.

System 10 and the method thereof and/or system 100 and the method thereof discussed above with reference to one or more of Figs. 1-7 may determine ICP similar as 17 disclosed in applicant's pending Published patent applications, U.S. Pub. No. 2016/0192849 published July 7, 2016 and U.S. Pub. No. 2016/0174858 published June 23, 2016 and applicant's U.S. Patent Nos. 9,826,913, 9,895,070, and 10,264,986, where the first output signals in the '849 Published Patent Application, the '858 Published Patent Application, the '913 Published Patent, the '070 Published Patent, and the '986 Patent correlate to the superficial output signals disclosed herein, the second output signals correlate to the corrected cranial output signals disclosed herein, and the third output signals correlate to the reference signals disclosed herein.

For example, similar as disclosed in the '849 Published Patent Application, in one example, ICP may be determined by determining the magnitude and phase of the spectral components of the first (superficial output signals), second (corrected cranial output signals), and third signals (reference signals) from each of first sensor, second sensor, and third sensor and comparing the magnitude or the phase of the spectral components of first sensor to the magnitude or the phase of the spectral components of third sensor and the magnitude or the phase of the spectral components of second sensor to the magnitude or the phase of the spectral components of third sensor and combining the compared values.

ICP may also be determined by combining the signals from the first sensor with the signals from the second sensor and combining the result with the signals from the third sensor. ICP may also be determined determining a first time lag between a peak of a signal from the first output signals (superficial output signals) to a peak of a signal from the third output signals (reference output signals) and a second time lag between a peak of a signal from the second output signals (corrected cranial output signals) to a peak of a signal from the third output signals (reference signals) and calculating the intracranial pressure based on the difference between the first time lag and the second time lag. ICP may also be determined by determining a time lag between a peak of a signal from the first output signals (superficial output signals) and a peak of signal from the second output signals (corrected cranial output signals) and calculating the intracranial pressure based on the time lag. ICP may also be determined by determining a first lag time between a peak of a signal from the first output signals (superficial output signals) to a peak of a signal from the third output signals (reference signals), a second time lag between a peak of a signal from the second output signals (corrected cranial output signals) to a peak of a signal from the third output signals (reference signals), and a third time lag between the peak of a signal from the first output signals (superficial output signals) to a peak of a signal from the second output signals (corrected cranial output signals) and calculating the intracranial pressure based on differences of the first, second, and third time lags.

Processing subsystem 40 shown in Figs. 1-7 may also include a feature extractor as disclosed in the '849 Patent Application configured to calculate one or more features from one or more of the first output signals (superficial output signals), the second output signals (corrected cranial output signals), and/or the third output signals (reference signals) and output the one or more features to an artificial neural network configured to calculate the intracranial pressure based on the one or more features. The feature extractor may be configured to calculate the one or more features individually from one or more of the first output signals (superficial output signals), the second output signals (corrected cranial output signals), and/or the third output signals (reference signals). The one or more features include one or more of: an amplitude of a largest peak in one of the first output signals (superficial output signals), the second output signals (corrected cranial output signals) and/or the third output signals (reference signals), a time from a beginning of a data acquisition cycle to a time of a maximum peak of one of the first output signals (superficial output signals), the second output signals (corrected cranial output signals) and/or the third output signals (reference signals), and a time between two different peaks of one of the first output signals (superficial output signals), the second output signals (corrected cranial output signals) and/or the third output signals (reference signals). The feature extractor may be configured to calculate the one or more features from a combination of signals of the first output signals (superficial output signals), the second output signals (corrected cranial output signals) and/or the third output signals (reference signals). The one or more features include one or more of: an amplitude of a largest peak in one of the first output signals (superficial output signals), the second output signals (corrected cranial output signals) and/or the third output signals (reference signals), a time from a beginning of a data acquisition cycle to a time of a maximum peak of one of the first output signals (superficial output signals), the second output signals (corrected cranial output signals) and/or the third output signals (reference signals), and a time between two different peaks of one of the first output signals (superficial output signals), the second output signals (corrected cranial output signals) and/or the third output signals (reference signals).

System 10, 100, and the method thereof shown in one or more of Figs. 1-7 may also include the artificial neural network as disclosed in the '849 Published Patent Application, which may be configured to combine the one or more features in a nonlinear fashion based on various weights in the structure of the artificial neural network to provide the intracranial pressure.

System 10, 100 and the method thereof may also calculate the phase shift of different frequencies included in the first output signals (superficial output signals) and the second output signals (corrected cranial output signals) and determine intracranial pressure of the human subject from the phase shift at the different frequencies of the first output signals (superficial output signals) and the second output signals (corrected cranial output signals). System 10, 100 may also calculate a difference between the first output signals (superficial output signals) and second output signals (corrected cranial output signals) and determine the intracranial pressure from the difference.

For enablement purposes only, the following code portions are provided which can be executed on one or more processors, a computing device, a computer, a smart device, or similar type device to carry out the primary steps and/or functions of the system and method for non-invasively measuring blood volume oscillations inside a cranium of a human subject discussed above with reference to one or more of Figs. 1-7. Other equivalent algorithms and code can be designed by a software engineer and/or programmer skilled in the art using the information provided herein:

```
%Acquire Cerebral signal
Get(Cer_NIR)
%Acquire Superficial signal
Get(Sup_NIR)
%Basic Data Conditioning of acquired signals
```

$$\text{Detrend\_Cer\_NIR} = \text{Det}(\text{Cer\_NIR})$$

$$\text{Detrend\_Sup\_NIR} = \text{Det}(\text{Sup\_NIR})$$

```
%Perform Frequency Domain Analysis:
```

$$\text{PSD\_Cer} = \text{fft}(\text{Detrend\_Cer\_NIR})$$

$$\text{PSD\_Sup} = \text{fft}(\text{Detrend\_Sup\_NIR})$$

```
%Identify Frequency Components of interest
```

$$\text{Filt\_Cer} = \text{FilterAtSpecificFreq}\,(\text{Detrend\_Cer\_NIR})$$

$$\text{Filt\_Sup} = \text{FilterAtSpecificFreq}\,(\text{Detrend\_Sup\_NIR})$$

```
%Use Superficial signal to remove External Contribution from Cerebral Signal
```

$$\text{Brain\_CleanSignal} = \text{Detrend\_Cer\_NIR} - \text{LeastSqaureFit}\,(\text{Filt\_Sup} : \text{Filt\_Cer}\,)$$

```
% This is just an example,
%several methods can be used to remove the external contribution from the cerebral signal
%Calculate Arrival Delay (or Pulse Transit Time: PTT) between Cerebral Signal and Reference Signal
```

$$InstantaneousPhase\_Brain\_CleanSignal = HilbertTrnaform(Brain\_CleanSignal)$$

$$InstantaneousPhase\_Filt\_Sup = HilbertTrnaform(Filt\_Sup)$$

$$PhaseDiff = InstantaneousPhase\_Brain\_CleanSignal - InstantaneousPhase\_Filt\_Sup$$

$$PTT = PhaseDiff \backslash (2*\pi*f);$$

% f is frequency associated to physiological parameter of interest

$$ICP = UseCalculatedPTTtoRetrieveICPfromLookUpTable;$$

% Look Up Table previously built based on empirical data

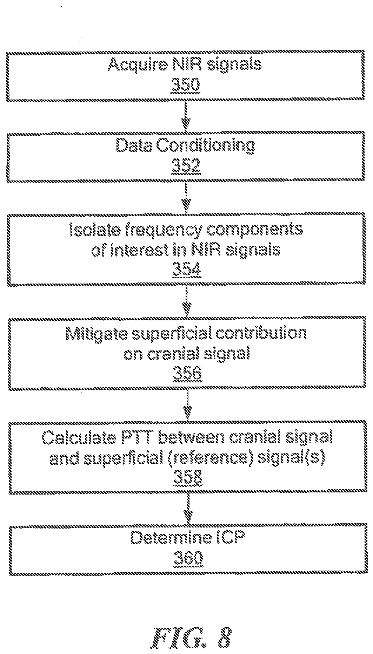

In this example, the exemplary code above executed by processing subsystem 40, of system 10, 100 and method thereof for non-invasively measuring blood volume oscillations inside a cranium of a human subject discussed above with reference to one or more of Figs. 1-7 preferably includes acquiring NIR signals, step 350, Fig. 8, data conditioning, step 352, isolating frequency components of interest in NIR signals, step 354, mitigating superficial contributions on cranial signals, step 356, calculating pulse transit time (PTT) between cranial signals and superficial (reference signal/s), step 358, and determine ICP, step 360.

Although specific features of the invention are shown in some drawings and not in others, this is for convenience only as each feature may be combined with any or all of the other features in accordance with the invention. The words "including", "comprising", "having", and "with" as used herein are to be interpreted broadly and comprehensively and are not limited to any physical interconnection. Moreover, any embodiments disclosed in the subject application are not to be taken as the only possible embodiments.

Other embodiments will occur to those skilled in the art and are within the following claims.

## Claims

1. A system (10) for non-invasively measuring blood volume oscillations inside a cranium (12) of a human subject (14), the system comprising:

   a first light source adapted (16) to be placed on the skin (17) above the cranium (12) of the human subject (14) configured to emit light (18) which penetrates a superficial space (20) outside the cranium (12);
   a detector (22) adapted to be placed on the skin (17) above the cranium (12) of the human subject (14) spaced from the first light source (16) by a first predetermined separation distance (d-24) that causes the detector (22) to detect light (18) which reflects from the superficial space (20) outside the cranium (12) and output superficial output signals;
   a second light source adapted (30) be placed on the skin (17) above the cranium (12) configured to emit light (32) which penetrates through the superficial space (20) outside the cranium (12) to inside the cranium (46) and the detector (22) is spaced from the second light source (30) by a second predetermined separation distance (d-34) that causes the detector (22) to detect light (32) which reflects from inside the cranium (46) and output cranial output signals;
   a processing subsystem (40) coupled to the first light source (16), the second light source (30), and the detector (22), the processing subsystem (40) configured to alternately enable the first light source (16) and the second light source (30) and alternately enable the detector (22) to detect the light (18) which reflects from the superficial space (20) outside the cranium (12) and the light (32) which reflects from inside the cranium (46) to generate the superficial output signals and cranial output signals, the processing subsystem (40) responsive to the superficial output signals and the cranial output signals and further configured to reduce contributions from the superficial space existing in the cranial output signals and generate corrected cranial output signals indicative of blood volume oscillations inside the cranium; and

   a reference sensor (50) adapted to be placed on the human subject (14) and coupled to the processing subsystem (40), the reference sensor (50) configured to provide reference signals for calculating a differential pulse transit time, a phase shift, or transfer function from one location on the human subject (14) to the corrected cranial output signals.

2. The system of claim 1 in which the first light source (16), the second light source (30), and the detector (22) are adapted to be placed on skin (17) above the cranium (12) of the human subject (14) with the first light source (16) located between the second light source (30) and the detector (22).

3. The system of claim 1 in which the first light source (16) and the second light source (30) are adapted to be placed on a forehead (60) of the cranium (12).

4. The system of claim 1 in which the reference sensor (50) is adapted to be placed on one of: an ear, a temple, a finger, a hand, a forearm, a wrist, a chest, a back, a leg, a toe, or foot of the human subject.

5. The system of claim 1 in which the reference sensor (50) is adapted to be placed on the human subject (14) approximately the same distance from a heart (70) of the human subject as the first light source (16) and the second light source (30).

6. The system of claim 1 in which processing system (40) is configured to calculate a phase shift of the corrected cranial output signals compared to the reference signals and determine intracranial pressure of the human subject (14) from the phase shift of the corrected cranial output signals compared to the reference signals.

7. The system of claim 1 in which processing system (40) is configured to calculate a difference or transfer function between the superficial output signals and the corrected cranial output signals and determine intracranial pressure from the difference or transfer function.

8. The system of claim 1 in which processing system (40) is configured to calculate a phase shift of the corrected cranial output signals compared to the superficial signals and determine intracranial pressure of the human subject from the phase shift of the corrected cranial output signals compared to the superficial signals.

9. The system of claim 1 in which the processing system (40) is configured to calculate a time delay between the corrected cranial output signals and the superficial output signals and determine intracranial pressure of the human subject from the time delay.

**Patentansprüche**

1. Ein System (10) zur nichtinvasiven Messung von Blutvolumenschwankungen in einem Schädel (12) eines menschlichen Individuums (14), wobei das System Folgendes umfasst:

eine erste Lichtquelle (16), die dazu angepasst ist, auf der Haut (17) über dem Schädel (12) des menschlichen Individuums (14) platziert zu werden, die dazu ausgelegt ist, Licht (18) abzugeben, das durch einen oberflächlichen Raum (20) außerhalb des Schädels (12) dringt;
einen Detektor (22), der dazu angepasst ist, auf der Haut (17) über dem Schädel (12) des menschlichen Individuums (14) platziert zu werden, der von der ersten Lichtquelle (16) durch einen ersten zuvor festgelegten Trennabstand (d-24) beabstandet ist, der bewirkt, dass der Detektor (22) von dem oberflächlichen Raum (20) außerhalb des Schädels (12) reflektiertes Licht (18) detektiert und oberflächliche Ausgangssignale ausgibt;
eine zweite Lichtquelle (30), die dazu angepasst ist, auf der Haut (17) über dem Schädel (12) platziert zu werden, die dazu ausgelegt ist, Licht (32) abzugeben, das durch den oberflächlichen Raum (20) außerhalb des Schädels (12) bis in das Innere des Schädels (46) dringt, und der Detektor (22) durch einen zweiten zuvor festgelegten Trennabstand (d-34) von der zweiten Lichtquelle (30) beabstandet ist, der bewirkt, dass der Detektor (22) aus dem Inneren des Schädels (46) reflektiertes Licht (32) detektiert und Schädel-Ausgangssignale ausgibt;
ein Verarbeitungs-Subsystem (40), das mit der ersten Lichtquelle (16), der zweiten Lichtquelle (30) und dem Detektor (22) gekoppelt ist, wobei das Verarbeitungs-Subsystem (40) dazu ausgelegt ist, abwechselnd die erste Lichtquelle (16) und die zweite Lichtquelle (30) zu aktivieren und abwechselnd den Detektor (22) zu aktivieren, um das von dem oberflächlichen Raum (20) außerhalb des Schädels (12) reflektierte Licht (18) und das aus dem Inneren des Schädels (46) reflektierte Licht (32) zu detektieren, um die oberflächlichen Ausgangssignale und Schädel-Ausgangssignale zu erzeugen, wobei das Verarbeitungs-Subsystem (40) auf die oberflächlichen Ausgangssignale und die Schädel-Ausgangssignale anspricht und ferner dazu ausgelegt ist, die Beiträge von dem oberflächlichen Raum, die in den Schädel-Ausgangssignalen existieren, zu reduzieren und korrigierte Schädel-Ausgangssignale zu erzeugen, die Blutvolumenschwankungen innerhalb des Schädels anzeigen; und
einen Bezugssensor (50), der dazu angepasst ist, auf dem menschlichen Individuum (14) platziert und mit dem Verarbeitungs-Subsystem (40) gekoppelt zu werden, wobei der Bezugssensor (50) dazu ausgelegt ist, Bezugssignale zur Berechnung einer Differenzial-Pulslaufzeit, einer Phasenverschiebung oder Übertragungsfunktion von einem Ort auf dem menschlichen Individuum (14) zu den korrigierten Schädel-Ausgangssignalen bereitzustellen.

2. System nach Anspruch **1,** wobei die erste Lichtquelle (16), die zweite Lichtquelle (30) und der Detektor (22) dazu angepasst sind, auf der Haut (17) über dem Schädel (12) des menschlichen Individuums (14) platziert zu werden, wobei sich die erste Lichtquelle (16) zwischen der zweiten Lichtquelle (30) und dem Detektor (22) befindet.

3. System nach Anspruch **1,** wobei die erste Lichtquelle (16) und die zweite Lichtquelle (30) dazu angepasst sind, auf einer Stirn (60) des Schädels (12) platziert zu werden.

4. System nach Anspruch 1, wobei der Bezugssensor (50) dazu angepasst ist, auf einem der Folgenden platziert zu werden: einem Ohr, einer Schläfe, einem Finger, einer Hand, einem Unterarm, einem Handgelenk, einem Brustkorb, einem Rücken, einem Bein, einem Zeh oder Fuß des menschlichen Individuums.

5. System nach Anspruch 1, wobei der Bezugssensor (50) dazu angepasst ist, auf dem menschlichen Individuum (14) ungefähr in dem gleichen Abstand von einem Herzen (70) des menschlichen Individuums wie die erste Lichtquelle (16) und die zweite Lichtquelle (30) platziert zu werden.

6. System nach Anspruch 1, wobei das Verarbeitungssystem (40) dazu ausgelegt ist, eine Phasenverschiebung der korrigierten Schädel-Ausgangssignale verglichen mit den Bezugssignalen zu berechnen und den Hirndruck des menschlichen Individuums (14) aus der Phasenverschiebung der korrigierten Schädel-Ausgangssignale verglichen mit den Bezugssignalen zu bestimmen.

7. System nach Anspruch 1, wobei das Verarbeitungssystem (40) dazu ausgelegt ist, eine Differenz oder Übertragungsfunktion zwischen den oberflächlichen Ausgangssignalen und den korrigierten Schädel-Ausgangssignalen zu berechnen und den Hirndruck aus der Differenz oder Übertragungsfunktion zu bestimmen.

8. System nach Anspruch 1, wobei das Verarbeitungssystem (40) dazu ausgelegt ist, eine Phasenverschiebung der korrigierten Schädel-Ausgangssignale verglichen mit den oberflächlichen Signalen zu berechnen und den Hirndruck des menschlichen Individuums aus der Phasenverschiebung der korrigierten Schädel-Ausgangssignale verglichen mit den oberflächlichen Signalen zu bestimmen.

9. System nach Anspruch 1, wobei das Verarbeitungssystem (40) dazu ausgelegt ist, eine Zeitverzögerung zwischen den korrigierten Schädel-Ausgangssignalen und den oberflächlichen Ausgangssignalen zu berechnen und den Hirndruck des menschlichen Individuums aus der Zeitverzögerung zu bestimmen.

**Revendications**

1. Système (10) de mesure non invasive des oscillations du volume sanguin à l'intérieur du crâne (12) d'un sujet humain (14), le système comprenant :

une première source lumineuse conçue (16) pour être placée sur la peau (17) au-dessus du crâne (12) du sujet humain (14), configurée pour émettre de la lumière (18) qui pénètre dans un espace superficiel (20) à l'extérieur du crâne (12) ;
un détecteur (22) conçu pour être placé sur la peau (17) au-dessus du crâne (12) du sujet humain (14), espacé de la première source lumineuse (16) d'une première distance de séparation prédéterminée (d-24) qui amène le détecteur (22) à détecter la lumière (18) qui se réfléchit à partir de l'espace superficiel (20) à l'extérieur du crâne (12) et à sortir des signaux de sortie superficiels ;
une deuxième source lumineuse conçue (30) pour être placée sur la peau (17) au-dessus du crâne (12), configurée pour émettre de la lumière (32) qui pénètre à travers l'espace superficiel (20) à l'extérieur du crâne (12) jusqu'à l'intérieur du crâne (46) et le détecteur (22) étant espacé de la deuxième source lumineuse (30) d'une deuxième distance de séparation prédéterminée (d-34) qui amène le détecteur (22) à détecter la lumière (32) qui se réfléchit à partir de l'intérieur du crâne (46) et à sortir des signaux de sortie crâniens ;
un sous-système de traitement (40) couplé à la première source lumineuse (16), à la deuxième source lumineuse (30) et au détecteur (22), le sous-système de traitement (40) étant configuré pour activer alternativement la première source lumineuse (16) et la deuxième source lumineuse (30) et permettre alternativement au détecteur (22) de détecter la lumière (18) qui se réfléchit à partir de l'espace superficiel (20) à l'extérieur du crâne (12) et la lumière (32) qui se réfléchit à partir de l'intérieur du crâne (46) pour générer les signaux de sortie superficiels et les signaux de sortie crâniens, le sous-système de traitement (40) répondant aux signaux de sortie superficiels et aux signaux de sortie crâniens et étant configuré en outre pour réduire les contributions provenant de l'espace superficiel existant dans les signaux de sortie crâniens et générer des signaux de sortie crâniens corrigés indiquant des oscillations du volume sanguin à l'intérieur du crâne ; et

un capteur de référence (50) conçu pour être placé sur le sujet humain (14) et couplé au sous-système de traitement (40), le capteur de référence (50) étant configuré pour fournir des signaux de référence pour le calcul d'un temps de transit d'impulsion différentielle, d'un déphasage, ou d'une fonction de transfert d'un emplacement sur le sujet humain (14) aux signaux de sortie crâniens corrigés.

2. Système selon la revendication 1, dans lequel la première source lumineuse (16), la deuxième source lumineuse (30) et le détecteur (22) sont conçus pour être placés sur la peau (17) au-dessus du crâne (12) du sujet humain (14), la première source lumineuse (16) étant située entre la deuxième source lumineuse (30) et le détecteur (22).

3. Système selon la revendication 1, dans lequel la première source lumineuse (16) et la deuxième source lumineuse (30) sont conçues pour être placées sur un front (60) du crâne (12).

4. Système selon la revendication 1, dans lequel le capteur de référence (50) est conçu pour être placé sur l'un d'une oreille, d'une tempe, d'un doigt, d'une main, d'un avant-bras, d'un poignet, d'une poitrine, d'un dos, d'une jambe, d'un orteil ou d'un pied du sujet humain.

5. Système selon la revendication 1, dans lequel le capteur de référence (50) est conçu pour être placé sur le sujet humain (14) approximativement à la même distance du cœur (70) du sujet humain que la première source lumineuse (16) et la deuxième source lumineuse (30).

6. Système selon la revendication 1, dans lequel le système de traitement (40) est configuré pour calculer un déphasage des signaux de sortie crâniens corrigés par rapport aux signaux de référence et déterminer la pression intracrânienne du sujet humain (14) à partir du déphasage des signaux de sortie crâniens corrigés par rapport aux signaux de référence.

7. Système selon la revendication 1, dans lequel le système de traitement (40) est configuré pour calculer une différence ou une fonction de transfert entre les signaux de sortie superficiels et les signaux de sortie crâniens corrigés et déterminer la pression intracrânienne à partir de la différence ou de la fonction de transfert.

8. Système selon la revendication 1, dans lequel le système de traitement (40) est configuré pour calculer un déphasage des signaux de sortie crâniens corrigés par rapport aux signaux superficiels et déterminer la pression intracrânienne du sujet humain à partir du déphasage des signaux de sortie crâniens corrigés par rapport aux signaux superficiels.

9. Système selon la revendication 1, dans lequel le système de traitement (40) est configuré pour calculer un temps de retard entre les signaux de sortie crâniens corrigés et les signaux de sortie superficiels et déterminer la pression intracrânienne du sujet humain à partir du temps de retard.

*FIG. 1*

FIG. 2

Processing
Subsystem
40

44

42

FIG. 3

FIG. 4

FIG. 5

120    104    106    122    12

**FIG. 6A**

120    104    12    106    122

**FIG. 6B**

120    104    12    106    122

**FIG. 6C**

120    104    12    106    122

**FIG. 6D**

Alternatively emitting light which penetrates a superficial space outside the cranium of the human subject
300

Alternately detecting the light which reflects from the superficial space outside the cranium of the human subject and outputting superficial output signals
302

Alternatively emitting light which penetrates inside the cranium of the human subject
304

Alternately detecting the light which reflects from inside the cranium of the human subject and outputting cranial output signals.
306

Responding to the superficial output signals and the cranial output signals and reducing contributions from the superficial space existing in the cranial output signals and generating output signals indicative of blood volume oscillations inside the cranium.
308

*FIG. 7*

```
┌─────────────────────────────────────┐
│         Acquire NIR signals         │
│                350                  │
└─────────────────────────────────────┘
                  │
                  ▼
┌─────────────────────────────────────┐
│         Data Conditioning           │
│                352                  │
└─────────────────────────────────────┘
                  │
                  ▼
┌─────────────────────────────────────┐
│     Isolate frequency components    │
│       of interest in NIR signals    │
│                354                  │
└─────────────────────────────────────┘
                  │
                  ▼
┌─────────────────────────────────────┐
│     Mitigate superficial contribution │
│          on cranial signal          │
│                356                  │
└─────────────────────────────────────┘
                  │
                  ▼
┌─────────────────────────────────────┐
│   Calculate PTT between cranial signal │
│   and superficial (reference) signal(s) │
│                358                  │
└─────────────────────────────────────┘
                  │
                  ▼
┌─────────────────────────────────────┐
│          Determine ICP              │
│                360                  │
└─────────────────────────────────────┘
```

*FIG. 8*

20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62973021
- US 20190269853 A
- US 20190021646 A
- US 20120108927 A
- WO 2019040849 A
- US 20180110449 A
- US 20150018697 A
- US 20060074283 A
- US 20160278643 A
- US 20090234245 A
- US 20160192849 A
- US 20160174858 A
- US 9826913 B
- US 9895070 B
- US 10264986 B